# EUROPEAN PATENT SPECIFICATION

(11) **EP 1 233 761 B1**
(45) Date of publication and mention of the grant of the patent: **28.03.2007**
(21) Application number: 00980663.9
(22) Date of filing: 21.11.2000
(51) Int. Cl.: A61K 9/24

(54) **OSMOTIC DOSAGE FORM COMPRISING FIRST AND SECOND COATS**
OSMOTISCHE DARREICHUNGSFORM MIT ZWEI MANTELSCHICHTEN
FORME POSOLOGIQUE OSMOTIQUE COMPRENANT UN PREMIER ET UN SECOND ENROBAGES

(30) Priority: 22.11.1999 US 166780 P
(43) Date of publication of application: 28.08.2002
(73) Proprietor: ALZA CORPORATION, Mountain View, CA 94039-7210 (US)
(72) Inventor: AYER, Atul, D., Palo Alto, CA 94303 (US); SHIVANAND, Padmaja, Mountain View, CA 94040 (US)
(74) Representative: Williams, Paul Edwin
(86) International application number: PCT/US2000/032065
(87) International publication number: WO 2001/037815

(56) References cited:
- EP-A- 1 025 845
- EP-A- 1 384 471
- WO-A-00/23055
- WO-A-96/00065
- WO-A-97/37640
- US-A- 4 743 248
- US-A- 4 795 644
- US-A- 5 916 595
- US-B- 5 472 710

## Description

### FIELD OF THE INVENTION

The present invention relates to a novel dosage form.

More particularly, the invention concerns a dosage form comprising means for delivering drugs that are difficult to deliver, which means functions in harmony with the dosage form to deliver a controlled-sustained release dose of drugs. The invention pertains also to a method of administering a controlled-sustained dose of drug to a patient in need of therapy.

### BACKGROUND OF THE INVENTION

In recent time, both controlled-drug delivery and sustained-release dosage forms have enjoyed popularity in the pharmaceutical and medical arts. The popularity for both of these dosage forms and their acceptance is attributed to their usefulness for delivering drugs that require a specific dose, and for the delivery of drugs at a non-toxicological rate of delivery. The controlled-delivery dosage forms seek to maintain a constant drug level in the blood, or in a target tissue for better therapy. The sustained-release dosage forms seek to provide drug delivery over an extended time for better therapy. The prior art indicates it is often desirable to control and maintain a sustained level of drug in the blood, or at a drug receptor site by using two different dosage forms to reach this goal. However, there are many drugs, for example acidic and basic drugs that do not lend themselves to delivery in the prior art dosage forms. While the prior art devoted attention to controlled delivery and to sustained release, there still is a pressing need for a novel dosage form that is capable of delivering drugs that do not lend themselves to delivery at a predetermined rate or a predetermined dose over a predetermined time. The goal of this invention, thus is to make available a dosage form that provides controlled-sustained release delivery of drugs that do not lend themselves to drug delivery from a single dosage form.

WO 97/37640A discloses a dosage form for providing a substantially uniform drug release rate. The dosage form comprises a drug composition, a semipermeable wall surrounding the drug composition, and an exit means in the wall for delivering the drug from the dosage form. The dosage form may further comprise an inner subcoat surrounding the drug composition for providing a drug free interval.

US 4,743,248 discloses a dosage form which comprises a beneficial agent formulation which forms a fluid environment having a pH of less than 5, and an inner wall which keeps its chemical integrity in a fluid environment having a pH of less than 5, but which loses its chemical integrity in a fluid environment having a pH of greater than 5.

WO 96/00065A discloses a dosage form for administering tacrine, which can comprise an inner coat between a tacrine composition and an outer semipermeable wall. The inner layer serves as a lubricating coat to facilitate delivery of tacrine from the dosage form.

US 5,916,595 discloses a dosage form which may comprise an optional inner coat of pH sensitive material, which dissolves in alkaline solution.

### SUMMARY OF THE INVENTlON

The present invention pertains to a novel dosage form comprising controlled-sustained release drug delivery over time. The dosage form comprises a pharmaceutical composition comprising a dose of drug, an inner membrane that surrounds the pharmaceutical composition, an outer membrane that surrounds the inner membrane and pharmaceutical composition, and a passageway that communicates with the therapeutic composition for delivering the drug to a drug receiving patient.

According to the present invention there is thus provided a dosage form comprising an acidic or basic drug formulation, an inner membrane surrounding the drug formulation, a displacement composition in contact with the inner membrane and an outer semipermeable membrane surrounding the inner membrane and the displacement composition, in which outer semipermeable membrane at least one exit passageway is formed; wherein the inner membrane comprises a pH sensitive membrane which is permeable to aqueous or biological fluid, and dissolves in the presence of either (i) an aqueous acidic drug formulation solution in the case that the dosage form comprises an acidic drug formulation or (ii) an aqueous basic drug formulation solution in the case that the dosage form comprises a basic drug formulation; and wherein the displacement composition in use imbibes an aqueous or biological fluid thereby expanding for displacing drug released by the inner membrane through the exit passageway in the outer membrane.

The dosage form of the invention comprising controlled-sustained release drug delivery provides important therapeutic advantages. The dosage form advantages comprise: controlled delivery of drug to provide a known constant dose over a sustained-release time; a sustained maintenance of a therapeutic blood level for an extended period of time; reduction of the frequency of dosing; increase in patient compliance in administered dose; lessening of undesired high drug concentrations administered to a patient; a nonenteric membrane to surround a drug in the dosage form; provide means for administering highly soluble acidic and basic drugs; provide means for preventing drug leakage from the dosage form; and means for concentrating a drug to avoid a descending drug release from the dosage form.

### DESCRIPTION OF THE INVENTION

The invention is made available by first providing a pharmaceutically acceptable composition. The composition comprises a drug selected from the group consisting of adrenergic agonist, adrenergic blocker, alcohol deterrent, aldose reductase inhibitor, anabolic, analgesic-narcotic, analgesic nonnarcotic androgen, anesthetic, anorexic, anthelmintic, antiacne, antiallergic, antiamebic, antiandrogen, antiarrhythmic, antiarteriosclerotic, antiarthritic, antirheumatic, antibacterial, antibiotic, anticholinergic, anticonvulsant, antidepressant, antidiabetic, antidiarrheal, antidiuretic, antiestrogen, antifungal, antiglaucoma, antigonadotropin, antigout, antihistaminic, antihyperlipoproteinemic, antihypertensive, antihyperthyroid, antihypotensive, antihypothyroid, anti-inflammatory, antimalarial, antimigraine, antinauseant, antineoplastic, anti-Parkinsonian, antipheochromocytoma, antipneumocystis, antiprostatic-hypertrophy, antiprotozoal, antirickettsial, antiseborrheic, antiseptic, antispasmodic, antithrombotic, antiulcerative, antiurolithic, antiviral, anxiolytic, benzodiazepine-antagonist, bronchodilator, calcium channel blocker, calcium regulator, cardiotonic, chelating agent, cholecystokinin antagonist, cholelitholyte-agent, choleretic, cholinesterase inhibitor, cholinesterase reactivator, decongestant, dental caries prophylactic, depigmentor, diuretic, dopamine receptor agonist, actoparasiticide, enzyme, estrogen, gastric secretin inhibitor, glucocorticoid, gonadotropic hormone, growth hormone inhibitor, growth hormone releasing factor, growth stimulant, hemolytic, heparin antagonist, hepatoprotectant, immunomodulation, immunosuppressant, lactation stimulating hormone, LH-RH agonist, lipotropic, lupus erythematosus suppressant, mineralocorticoid, monoamine oxidase inhibitor, mucolytic, muscle relaxant, narcotic antagonist, neuroprotective, ovarian hormone, oxytocic, pepsin inhibitor, persistaltic stimulant, progestin, prolactin inhibitor, prostaglandin, prostaglandin analog, protease inhibitor, respiratory stimulant, sclerosing agent, sedative-hypnotic, thrombolytic, thyrotropic hormone, uricosuric, vasodilator, vasoprotectant, vitamin, and vulnerary.

The drug present in the pharmaceutical composition is present either as an acid or as a base. For the purpose of this invention an acid is a drug capable of yielding a proton (hydrogen ion), and a base is a drug capable of accepting a proton. Also, for the purpose of this invention, a drug may be described by its pKₐ or pK_{b} value that numerically compares the relative acidity or basicity of ionizing drugs in solution. An acid drug for this invention exhibits a pH of greater than zero usually 1 to 6, and a basic drug exhibits a pH of 8 to 14. Representative of drug that exhibit a pH of 1 to 6 are venalfaxine hydrochloride, tripelennamine hydrochloride, tripelennamine citrate, oxytetracycline hydrochloride, tetracycline hydrochloride, metronidazole hydrochloride, oxyphenonium hydrobromide, phenoxybenzamine enitabas palmitate, aminocaproic acid, disopyramide hydrosulfate, hydrophosphate, verapamil hydrochloride, papaverine tartrate, fluphenazine hydrochloride, ascorbic acid, prochlorperazine edisylate, citric acid, fluphenazine edelate, tetracycline fumarate, hydroxyzine lactate, benzaquinamide stearate and thiamine ascorbate; representative of drugs that exhibit a pH of 8 to 14 are ozatadine maleate, chlorpheniramine maleate, doxylamine succinate, promethazine, clindamycin phosphate, neostigmine bromide, homatropine methylbromide, bitolterol mesylate, ephedrine, phenylpropanolamine, timolol maleate, codeine phosphate, phenobarbital sodium, phenytoin sodium, dextroamphetamine, amylobarbitone sodium, butabarbital sodium, colchicine, and pralidoxine chloride. Other drugs that may be delivered by the dosage form of the invention include sodium acyclovir, sodium valproate, clindamycin hydrochloride, tramado hydrochloride, oxybutynin hydrochloride, oxtriphylline hydrochloride. The pK values of medicinals are disclosed by Newton and Kluza in Drug Intelligence and Clinical Pharmacy, Vol. 12, pp 546-554, (1978); the pH values of medicinals are disclosed in EP 377518-B1; and procedures for ascertaining pK and pH are disclosed in Pharmaceutical Sciences, by Remington, 17^{th} Ed., pp 234-248, (1985).

The drug in the pharmaceutical composition can be present with compositional-forming ingredients, such as a binder, lubricant, wetting agent, osmagent, and osmopolymer. Representative binders comprise 0 ng to 30 mg, usually 1 ng to 30 mg of a member selected from the group consiting of acacia, alginic acid, ethylcellulose, gelatin, guar gum, hydroxyethylcellulose, hydroxypropylcellulose and hydroxypropylmethylcellulose; representative lubricants comprise 0 ng to 10 mg, generally 1 ng to 10 mg of a member selected from the group consisting of sodium lauryl sulfate, fatty amines and fatty quaternary ammonium salts; representative antioxidants comprise 0 ng to 10 mg, generally 1 ng to 10 mg of a member selected from the group consisting of alpha tocopherol, ascorbic acid, ascorbyl palmitate, butylated hydroxyanisole, butylated hydroxytoluene, and sodium ascorbate; and representative coloring agent comprise 0 ng to 5 mg, generally 1 ng to 5 mg of a coloring agent selected from the group consisting of tartazine, erythrosine, titanium dioxide, iron oxides, FD&C blue, FD&C red, D&C orange, and D&C yellow; viscosity-increasing agents comprising 0 ng to 200 mg, generally 1 ng to 200 mg of a member selected from the group consisting of methylcellulose, polyvinyl alcohol, polyvinyl pyrrolidone, propylene glycol alginate, starch, tragacanth and colloidal silicon dioxide. The pharmaceutical composition forming ingredients are disclosed in the Handbook of Pharmaceutical Excipients, Edited by Wade & Weller; 1994.

The pharmaceutical composition comprises an osmagent, when used according to the mode of the invention wherein the pharmaceutically acceptable drug is not itself osmotically active. The osmagent, also known as osmotic solute and osmotically effective compound, is soluble in aqueous and biological fluids that enters the dosage form and it exhibits an osmotic pressure gradient across the semipermeable wall against an exterior fluid. Osmotically effective osmagents useful for the present purpose comprise magnesium sulfate, magnesium chloride, sodium chloride, lithium chloride, potassium sulfate, sodium carbonate, sodium sulfite, lithium sulfate, potassium chloride, sodium sulfate, mannitol, urea, sorbitol, inositol, raffinose, sucrose, glucose, and fructose. The osmagent is present in an excess amount, comprising 5 mg to 210 mg, and it can be in any form such as particles, granules, and the like. The osmotic pressure in atmospheres of the osmagents for this invention will be greater than zero and up to 5.07 X 10⁷ Nm⁻² (500 atm) or higher. The osmagents are known in U.S. Patent No. 5,248,310 issued to Barclay et al; and in U.S. Patent No. 4,783,337 issued to Wrong et al.

The pharmaceutical composition can comprise an osmopolymer. The osmopolymers are hydrophillic polymers that interact with water and aqueous biological fluids, whereby they swell or expand in the presence of the imbibed fluids. The osmopolymer in operation serves as a pharmaceutically acceptable carrier for delivering a drug from the dosage form. The osmopolymers formulated with a drug to provide the pharmaceutically acceptable composition comprise a member selected from the group consisting of poly(olefin), poly(vinyl), poly(addition), poly(condensation), poly(erodible), and poly(hydrogel), as represented by poly(alkylene oxide) of 50,000 to 1,500,000 weight average molecular weight including poly(ethylene oxide) of 100,000 molecular weight, poly(ethylene oxide) of 200,000 molecular weight, poly(ethylene oxide) of 100,000 molecular weight blended with poly(propylene oxide) of 300,000 molecular weight; a carboxyalkylcellulose of 10,000 to 1,500,000 weight average molecular weight such as sodium carboxymethylcellulose, potassium carboxymethylcellulose, lithium carboxyethylcellulose, alkali carboxymethylcellulose, and sodium carboxyethylcellulose. The pharmaceutical composition comprises 10 mg to 250 mg of the osmopolymer. The osmopolymers are disclosed in U.S. Patent No. 5,840,754 issued to Guittard et al; and in U.S. Patent No. 5,248,310 issued to Barclay et al.

The pharmaceutical composition is surrounded or enveloped by an inner membrane that is permeable to the passage of aqueous and biological fluids and is soluble in either aqueous acidic solution or aqueous basic solution. These aqueous acidic and/or basic solutions are formed in situ by an acidic drug or a basic drug dissolving and forming a corresponding solution that dissolves the inner membrane over time. Representative of polymers for manufacturing the inner membrane comprise a member selected from the group consisting of poly(methacrylates), poly(methacrylic acid methyl methacrylate), poly(2-vinylpyridine), poly(4-vinylpyridine), poly(N-pyrrolidonylethylene), poly(iminoadipolylimeno-trimethylene-1,4-phenylinetrimethylene), poly(iminoadipolylimenotrimethylene-1,4-phenylineposphinedose) poly(diethylcarbodimide), poly(1-4 piperazinediylcarbonyl-ethylene-1,4-phenylenephosphinylidene), poly(1,4-phenyl-enethylenecarbonylidene); and poly(2-vinylpyridine-co-styrene). The membrane surround the pharmaceutical composition is 0.1 mil (0.0003 cm) to 10 mil (0.0254 cm). The inner membrane can be neuroporous by incorporating therein an aqueous leachable compound such as glucose, fructose, urea and the like. The polymers are known in Polymer Handbook, 2^{nd} Ed., pp IV-242-IV-262, (1975) edited by Brandrup and Immergut, published by John Wiley and Sons; and Handbook of Pharmaceutical Excipients, 2^{nd} Ed., edited by Wade and Weller; pp 362-365, (1994), published by The Pharmaceutical Press.

The inner membrane surrounding the pharmaceutical composition is surrounded by an outer membrane. The outer membrane comprises totally, or in part, a composition that is permeable to the passage of an exterior fluid present in the biological environment, including humans, of use. The outer membrane is impermeable to the passage of drug. The outer semipermeable membrane is inert and it maintains its physical and chemical integrity during the delivery of a drug from the dosage form. The outer membrane is formed of a member selected from the group consisting of a cellulose, cellulose, ether, cellulose ester and a cellulose ester-ether. The cellulose polymers comprise a degree of substitution. D.S., on the anhydroglucose unit, from greater than 0 up to 3 inclusive. By degree of substitution is meant the average number of hydroxyl groups originally present on the anhydroglucose unit comprising the cellulose polymer that are replaced by a substituting membrane-forming group. Representative materials for forming the membrane include a member selected from the group consisting of cellulose acylate, cellulose diacylate, cellulose triacylate, cellulose acetate, cellulose diacetate and cellulose triacetate. The cellulose polymers include mono, di and tricellulose, alkanylates, alkenylates, and mono, di and tricellulose aroylates. Exemplary polymers include cellulose acetate having a D.S. up to 1 and an acetyl content up to 21 %; cellulose acetate having an acetyl content of 32 to 39.8%; cellulose acetate having a D.S. of 1 to 2 and an acetyl content of 21 to 35%; and cellulose acetate having a D.S. of 2 to 3 and an acetyl content of 35 to 44.8%. More specific cellulose polymers include cellulose propionate having a D.S. of 1.8 and a propyl content of 39.2 to 45% and a hydroxyl content of 2.8 to 5.4%; cellulose acetate butyrate having a D.S. of 1.8% and an acetyl content of 13 to 15% and a butyryl content of 34 to 39%, cellulose acetate butyrate having an acetyl content of 2 to 29%, a butyrl content of 17 to 53% and a hydroxyl content of 0.5 to 4.7%; cellulose triacylates having a D.S. of 2.9 to 3% such as cellulose triacetate, cellulose trivalerate, cellulose trilaurate, cellulose tripalmitate, cellulose trisuccinate, and cellulose trioctanoate; cellulose diacylates having a D.S. of 2.2 to 2.6% such as cellulose disuccinate, cellulose dipalmitate, cellulose diotanoate, cellulose dipentanoate; and coesters of cellulose such as cellulose acetate butyrate, cellulose acetate propionate, cellulose isopropyl acetate, and cellulose acetate pentanoate.

Additional semipermeable polymer for forming the outer membrane comprise acetalaldehyde, dimethylcellulose, cellulose acetate ethyl carbamate, cellulose acetate methyl carbonate, cellulose acetate methyl carbamate, cellulose acetate dimethyl aminoacetate, semipermeable polyamides; semipermeable polyurethanes; semipermeable sulfonated polystyrenes; semipermeable cross-linked selectively permeable polymers formed by the coprecipitation of a polyanion and a polycation as disclosed in U.S. Pat. Nos. 3,173,876; 3,276,586; 3,541,005; 3,541,006; 3,546,142; 4,950,486; 4,966,769; and 5,057,321; semipermeable polymers as disclosed by Loeb and Souriajan in U.S. Pat. No. 3,133,132; semipermeable lightly cross-linked polystyrene derivatives; semipermeable cross-linked poly(sodium styrene sulfonate); and semipermeable cross-linked poly(vinylbenzyltrimethyl ammonium chloride). The polymers are known in U.S. Pat. Nos. 3,845,770; 3,916,899; 4,160,020; and 5,358,721; and in the *Handbook of Common Polymers,* by Scott, J R and Roll, W J, 1971, published by CRC Press, Cleveland, Ohio.

The outer membrane, in another manufacture, comprises 0 weight percent to 35 weight percent, generally 1 weight percent to 35 weight percent of a polyethylene glycol comprising a member selected from the group consisting of a polyethylene glycol having a 200 to 900 molecular weight as a thick viscous liquid, and a polyethylene glycol having a 900 to 8000 molecular weight as a waxy solid. The polyethylene glycols are blended with the cellulose membrane-forming polymer to increase the flexibility of the membrane, to decrease the brittleness of the membrane, and to increase aqueous fluid-flux through the membrane. The polyethylene glycol also decreases the surface tension of the membrane, thereby increasing the coating of the membrane around the inner membrane, by increasing wetting properties of the outer membrane. The polyethylene glycols are known in Remington's Pharmaceutical Sciences, 17^{th} Ed., p. 1305 (1985) published by Mark Publishing Co., Easton, PA. Other wall forming ingredients include copolymers of polyethylene - propylene glycol as disclosed in Handbook of Pharmaceutical Excipients, 2^{nd} Ed., edited by Wade and Weller, (1994).

The invention provides a dosage form for both the controlled and sustained delivery of a drug up to thirty hours. The dosage form comprises an exit means in the outer membrane for delivering a dose of drug from the dosage form. The expression "exit means" comprises means and methods through the outer membrane for the controlled-metered delivery and metered-sustained release of drug from the dosage form. The dosage form comprises one or more exit means in the outer membrane. The exit means may comprise an aperture, orifice, bore, pore, porous element, hollow fiber, capillary tube, porous insert and porous overlay. Exit means includes a composition that erodes or is leached from the membrane in a fluid environment of use to produce exit means in dosage form. Representative materials suitable for forming an exit passageway or a multiplicity of exit passageways include erodible poly(glycolic) acid or poly(lactic acid) in gelatinous filament, polyvinyl alcohol, leachable materials such as fluid removable pore forming polysaccharides like glucose salts or oxides. A passageway or a plurality of passageways can be formed by leaching a material such as sorbitol from the membrane. The passageway can have any shape such as round, triangular, square, elliptical and other geometric shapes. Also, the dosage form can have more than one passageway in spaced apart relationship, or the exit means can be on a single surface or on more than one surface of the dosage form. Exit means equipment for forming exit means are disclosed in U.S. pat. Nos. 3,845,770; 3,916,899; 4,063,064; 4,088,864 and 4,950,486. Passageways formed by leaching are disclosed in U.S. Pat. Nos. 4,200,098; 4,285,987; and in 5,358,721.

The dosage form provided by the invention comprises a displacement composition. The displacement composition is surrounded by the outer membrane and it displaces or pushes drug released by the inner membrane through the exit means from the dosage form. The displacement composition is positioned in contact with the inner membrane pharmaceutical formulation. The displacement composition comprises an osmopolymer such as 20 mg to 450 mg of a poly(alkylene oxide) comprising a 2,000,000 to 10,000,000 weight average molecular weight, comprising poly(ethylene oxide) of 2,500,000 molecular weight, poly(ethylene oxide) of 5,000,000 molecular weight, poly(ethylene oxide) of 7,000,000 molecular weight, poly(ethylene oxide) of 7,800,000 molecular weight, and poly(propylene oxide) of 4,250,000 molecular weight; osmopolymers acrylic acid cross-linked with a polyalkylsucrose, also, known as carboxypolymethylene and as carboxyvinyl polymer comprising a weight average molecular weight of 125,000 to 7,500,000; polysaccharides represented by xantham gum, polyhexoses, polypentoses, and dextrin of 10,000 to 4,500,000 molecular weight; and alkali carboxyalkylcellulose of 10,000 to 8,500,000 weight average weight comprising sodium carboxymethylcellulose, potassium carboxymethylcellulose, lithium carboxyethylcellulose, and calcium carboxybutylcellulose. The osmopolymers are disclosed in U.S. Pat. Nos., 5,160,744; 5,358,721; and 5,620,705; and in Handbook of Common Polymers, by Scott and Roff, published by the Chemical Company, Cleveland, Ohio; ACS Symposium Series, No. 31, by Ratna and Hoffman, pp 1-36, (1976) published by the American Chemical Society; and in Recent Advances in Drug Delivery Systems, by Schnacht, pp 259-278, published by Plenum Press; NY.

The displacement composition comprises from 100 ng (nanogram) to 35 mg of an osmagent for imbibing aqueous/biological fluid into the dosage form, that cooperates with the osmopolymer for displacing or pushing a drug from the dosage form. Osmagents effective for this, purpose comprise a member selected from the group consisting of magnesium sulfte, magnesium phosphate, magnesium chloride, magnesium bromide, sodium chloride, lithium chloride, potassium sulfate, sodium carbonate, sodium sulfite, lithium sulfate, potassium chloride, mannitol, urea, sorbitol, inositol, raffinose, sucrose, glycose, and glucose. The osmagent is usually present in an excess of its solubility in imbibed fluid, and it can be any form including particle, powder, granules and crystal. The osmotic pressure in atmospheres of the osmagent for the purpose of this invention will be greater than zero up to 5.07 X 10⁷ Nm-² (500 atmospheres (atm)) of osmotic pressure or higher. The osmagents are known in the U.S. Pat No. 5,053,032 issued to Barclay et al. The displacement composition comprises 0.1 mg to 10 mg of a lubricant such as magnesium stearate and stearic acid.

The dosage form provided by the invention is manufactured by conventional techniques, comprising in one manufacture the following procedure. The drug and formulation ingredients are mixed into a solid, semisolid or gel by ballmilling, calendering, stirring or rollmilling and then pressed into a preselected shape. The mixing can be achieved in the presence of a solvent or dry. Next, an inner membrane is applied to surround around the inner drug formulation. The inner membrane is applied by molding, spraying, dripping, or by air-suspension coating procedures. The outer membrane is applied around the inner membrane by like techniques comprising molding, spraying, dripping, or by air suspension techniques. An exit passageway is laser, or mechanically drilled through the outer membrane.

The displacement composition is manufactured into the dosage form by placing it in layered arrangement next to the inner membrane that surrounds the drug formulation. Next, the two layers comprising the displacement layer and the inner drug formulation layer are pressed together by using a conventional two-layer tablet press. The outer membrane next is applied by molding, spraying, dripping, or coating by air suspension techniques. The air suspension procedure consists in suspending and tumbling the two layered core in a current of air comprising a membrane forming composition surrounds the bilaminate core. The air suspension procedure is described in U.S. Pat. No. 2,799,241; J Am Pharm Assoc, Vol. 48, pp 451-459 (1959); J Am Pharm , Vol. 49, pp 82-84 (1960). Other manufacturing procedures are described in Modern Plastics Encyclopedia, Vol. 46, pp 62-70 (1960); and in Pharmaceutical Sciences, by Remington, 14^{th} Ed., pp 1626-1978(1970)..

Exemplary solvents suitable for manufacturing the drug formulation, the displacement layer, the inner membrane and the outer membranes comprise inert inorganic and organic solvents that do not adversely harm the materials comprising the dosage form. The solvents broadly include members selected from the group consisting of aqueous solvents, alcohols, ketones, esters, ethers, aliphatic hydrocarbons, halogenated solvents, cycloaliphatics, aromatics, heterocyclic solvents and mixtures thereof. Typical solvents include acetone, diacetone alcohol, methanol, ethanol, isopropyl alcohol, butyl alcohol, methyl acetate, methyl isobutyl ketone, methyl propyl ketone, n-hexane, n-heptane, ethylene glycol monoethyl ether, ethylene glycol monoethyl acetate, methylene dichloride, ethylene dichloride, propylene dichloride, carbon tetrachloride, chloroform, nitroethane, nitropropane, tetrachloroethane, ethyl ether, isopropyl ether, cyclohexane, cyclooctane, benzene, toluene, naphtha, 1,4-dioxane, tetrahydrofuran, diglyme, aqueous and nonaqueous mixtures thereof, such as acetone and water, acetone and methanol, acetone and ethyl alcohol, methylene dichloride and methanol, and ethylene dichloride and methanol.

### EXAMPLES

The following examples are merely illustrative of the present invention and they should not be considered as limiting the scope of the invention in any way as these examples and other equivalents thereof will become apparent to those versed in the art in the light of the present disclosure and the accompanying claims.

### EXAMPLE 1

A dosage form for administering the drug venlafaxine hydrochloride is manufactured as follows: first, 95 g of venlafaxine hydrochloride is mixed with 5 g of binder poly(vinylpyrrolidone) in a standard mixer. Next, ethanol is added to the blender while mixing until granules are formed in the mixer. The wet granules next are dried at room temperature 21°c (70°f) . Then, the granules are passed through a 20 mesh screen and then lubricated with 0.25 g of magnesium stearate. Next, the lubricated granules are poured into the die cavities of a 9/32 inch (0.714 cm) punch and compressed into standard drug cores.

Next, a polymer suitable for forming an aqueous permeable membrane, such as, for example poly(2-vinylpyrridine-co-styrene), is dissolved in acetone. The polymer content in solution is maintained at 5 weight %. The compressed cores then are placed into a 12 inch (30 cm) coating pan and the polymer solution is applied to the cores using standard solvent coating parameters. The solution is applied until 10 mg of membrane material surrounds the core, thereby providing the inner membrane surrounding the drug formulation.

Next, a semipermeable membrane is used to envelope the inner membrane drug formulation. The semipermeable cellulose membrane comprises cellulose acetate comprising 39.8% acetyl content and a poly(ethylene glycol) flux enhancer (95:5 v:v) dissolved in cosolvent mixture comprising acetone and water. The tablets are coated with a 22 mg coat. Then, the dual membrane coated tablets comprising the outer and inner membrane are drilled with a 30 mils (0.76 mm) exit orifice and dried in an oven at 45°C, to provide the dosage form. The dosage form administers a controlled-sustained dose of venlafaxine hydrochloride for 24 hours.

### EXAMPLE 2

The procedure of Example 1 is followed in this example with the conditions as set forth, except that venlafaxine hydrochloride is replaced by a water soluble acidic member selected from the group consisting of amitriptylene hydrochloride in an amount to provide a dose of 25 mg to 150 mg over 24 hours, clindamycin hydrochloride in an amount to provide a dose of 75 mg to 600 mg over 24 hours, tramadol hydrochloride in an amount to provide a dose of 5 mg to 200 mg over 24 hrs, oxybutynin hydrochloride in an amount to provide a dose of 1 mg to 30 mg over 24 hrs, and oxtriphylline hydrochloride in an amount to provide a controlled-sustained dose of 100 mg to 600 mg over 24 hours.

### EXAMPLE 3

A dosage form for the controlled-sustained delivery of the basic drug sodium phenytoin is manufactured as follows: first, 70 g of sodium phenytoin is mixed in a planetary mixer with 10 g of tromethamine and 5 g of poly(vinylpyrrolidone) at 70°F (21 °C). Next, ethanol is added to the mixer while mixing until granules are formed. Next, the wet granules are dried at 20°C, passed through a 20 mesh screen and then blended with 0.25 weight% magnesium stearate. Then, the lubricated granules are fed into a 15/32 inch (1.19 cm) die cavity punch and compressed into drug cores.

The compressed drug cores next are surrounded with an internal membrane. The internal membrane represented by polymethacrylates cationic and anionic polymers of dimethylaminoethylmethacrylate, methacrylic acid and methacrylic acid esters. The drug cores, in this example, are enveloped with Eudragit^{®} L 100, a poly(methacrylic acid, methyl methacrylate) 1:1, dissolved in isopropyl alcohol to give a solid content of 12 weight%. The compressed cores are placed into a 12 inch (30 cm) coating pan and the polymer solution coated around the cores. The polymer solution is applied to yield a 30 mg weight gain.

Next, an outer membrane is coated around the inner membrane coated drug core. The outer membrane comprises cellulose acetate comprising an acetyl content of 39.8% and a flux enhancer polyethylene-propylene glycol copolymer (85:15 wt:wt), dissolved in a cosolvent comprising acetone and water. The coating is applied to achieve a 50 mg weight gain. The dual membrane coated dosage forms are drilled with a 30 mil (0.76 mm) exit orifice and dried in an oven at 45°C to provide the dosage form.

### EXAMPLE 4

The procedure of Example 3 is followed with the conditions as set forth, except the drug in this example is sodium valproate.

### EXAMPLE 5

The procedure of Example 1 is followed in this example with the addition of a displacement composition manufactured into the dosage form. The displacement composition is prepared by screening 185 g of polyethylene oxide having a 7,500,000 molecular weight through a 40 mesh screen, and then blending the polyethylene oxide with 50 g of the osmagent sodium chloride. Next, the fresh prepared mixture is mixed with 12.5 g of polyvinylpyrrolidone having a 60,000 molecular weight and with 1.25 g of colorant ferric oxide. Then, the ingredients are mixed for 5 minutes and 300 ml of denatured anhydrous ethanol is added slowly to the blending mixture and all the ingredients blended for an additional 5 minutes. The wet mixture is passed through a 20 mesh screen and allowed to dry at room temperature for 18 hours. The dry blend is screened again through a 20 mesh screen. The screened mixture is mixed with 1.25 g of magnesium stearate.

Next, the inner membrane drug composition, as prepared in Example 1, weighing 500 mg is added to a press, then 250 mg of the displacement composition is added to the press, and the two distinct and contacting compositions are pressed under a pressure of 1633kg (1.8 tons) to yield a bilayered composition.

Then, the bilayered drug and displacement core is surrounded with a semipermeable wall comprsing 80 wt% cellulose acetate having an acetyl content of 39.8% and 20 wt% of aqueous flux transport polyethylene glycol having a molecular weight of 3350. The semipermeable membrane forming composition is dissolved in methylene chloride: methanol (85:15 wt:wt) cosolvent to make a 4 weight% solids solution. The semipermeable outer membrane composition is sprayed onto and around the bilayered pressed arrangement in an air suspension coater. Then, the dosage form is dried for 24 hours at room temperature. Then, two exit passageways 0.635 mm are laser driled through the semipermeable membrane facing the inner membrane drug composition. Any residual solvent is removed by drying the dosage form for 40 hours at 50°C and at a 50% relative humidity. The osmotic dosage form exhibits a controlled-sustained release rate over 20 hrs.

### EXAMPLE 6

A dosage form for administering sodium acyclovir for treating viral infected patients in need of antiviral therapy is prepared as follows: first, 116.25 of polyethylene oxide having a 100,000 molecular weight is screened through a 40 mesh screen, then 116.25 g of a different polyethylene oxide having a 300,000 molecular weight is screened through a 40 mesh screen, and then 15 g of hydroxypropylmethylcellulose of 11,200 molecular weight is screened through a 40 mesh screen, and the three screened ingredients are placed into a beaker. Next, 250 g of sodium acyclovir is added to the beaker, the ingredients mixed, transferred to a closed jar, and roll mixed for 25 minutes to effect a homogenous mix. Then, the dry mix is wetted with 275 ml of anhydrous ethanol, mixed again, passed through a 20 mesh screen and air dried to let the ethanol evaporate from the mixture. After drying, the dry mixture is screened through a 20 mesh screen to provide dry granules, to which is added 2.5 g of magnesium stearate. The final mixture is roll mixed for 5 minutes to produce a homogenous blend. Next, 500 mg of the sodium acyclovir formulation is compressed into 9/32 inch (0.714 cm) layers in a press. The individual layer are next coated with poly(methacrylic acid, methyl methacrylate) 1:2 soluble at >pH 7 and commercially available from Rohm Pharma, Germany.

Next, the membrane coated sodium acyclovir formulation is positioned next to the displacement composition prepared in Example 5 and the two layers are pressed under a pressure of two tons to produce an osmotic bilayer core. The core is then surrounded with a semipermeable cellulose membrane as prepared in Example 5. The dosage form dispenses sodium acyclovir at a controlled and sustained release rate for a prolonged period up to 20 hrs.

### PRACTICING THE INVENTION

The present invention provides a dosage form for delivering acidic drugs and basic drugs. The invention uses pH sensitive, acid or base, polymers that function structurally as a membrane surrounding a drug that produces an acidic or basic environment in the presence of an aqueous or biological fluid. This environment comprising the dissolved drug solution gradually dissolves the pH sensitive membrane attributing to the controlled and sustained release of the drug. The novel osmotic dosage forms of this invention uses dual means comprising an outer cellulosic membrane and an inner noncellulose membrane for the attainment of controlled and sustained release rates of drugs that are different to deliver for an indicated therapy.

A method of using the invention comprises (A) admitting orally into a patient in need of therapy, a dosage form comprising: (1) a semipermeable outer cellulosic membrane that surround an inner semipermeable noncellulose membrane which inner membrane envelopes a member selected from the group consisting of an acidic and basic drug; (2) an exit in the outer membrane for delivering the drug from the dosage form; (B) imbibing fluid through the outer and inner membranes causing the drug to slowly dissolve the inner membranes and develop an osmotic-hydrostatic presence whereby; (C) drug is delivered through the exit at a controlled-sustained rate over a prolonged time.

The dosage form also comprises:
(3) a displacement-push composition that imbibes an aqueous or a biological fluid thereby expanding for pushing the drug through the exit at a controlled sustained rate over time.

## Claims

1. A dosage form comprising an acidic or basic drug formulation, an inner membrane surrounding the drug formulation, a displacement composition in contact with the inner membrane and an outer semipermeable membrane surrounding the inner membrane and the displacement composition, in which outer semipermeable membrane at least one exit passageway is formed; wherein the inner membrane comprises a pH sensitive polymer membrane which is permeable to aqueous or biological fluid, and dissolves in the presence of either (i) an aqueous acidic drug formulation solution in the case that the dosage form comprises an acidic drug formulation or (ii) an aqueous basic drug formulation solution in the case that the dosage form comprises a basic drug formulation; and wherein the displacement composition in use imbibes an aqueous or biological fluid thereby expanding for displacing drug released by the inner membrane through the exit passageway in the outer membrane.

2. A dosage form according to claim 1 wherein the inner membrane dissolves in the presence of an acidic drug formulation solution.

3. A dosage form according to claim 1 wherein the inner membrane dissolves in the presence of a basic drug formulation solution.

4. A dosage form according to claim 1 wherein the outer membrane comprises a cellulosic polymer and the inner membrane comprises a non-cellulosic polymer.

5. A dosage form according to claim 4 wherein the inner membrane comprises a polymer selected from the group consisting of poly(methacrylates), poly(methacrylic acid methyl methacrylate), poly(2-vinylpyridine), poly(4-vinylpyridine), poly(N-pyrrolidonylethylene), poly(iminoadipolylimeno-trimethylene-1,4-phenylinetrimethylene), poly(iminoadipolylimenotrimethylene-1,4-phenyline-posphinedose) poly(diethylcarbodimide, poly(1,4- piperazinediylcarbonyl-ethylene-1,4-phenylenephosphinylidene), poly(1,4-phenyl-enethylenecarbonylidene); and poly(2-vinylpyridine-co-styrene).

6. A dosage form according to claim 5 wherein the drug is selected from the group consisting of venlafaxine hydrochloride, clindamycin hydrochloride, tramadol hydrochloride, oxybutynin hydrochloride, oxtriphylline hydrochloride, tripelennamine hydrochloride, tripelennamine citrate, oxytetracycline hydrochloride, tetracycline hydrochloride, metronidazole hydrochloride, oxyphenonium hydrobromide, phenoxybenzamine enitabas palmitate, aminocaproic acid, disopyramide hydrosulfate, hydrophosphate, verapamil hydrochloride, papaverine tartrate, fluphenazine hydrochloride, ascorbic acid, prochlorperazine edisylate, citric acid, fluphenazine edelate, tetracycline fumarate, hydroxyzine lactate, benzaquinamide stearate, thiamine ascorbate, ozatadine maleate, chlorpheniramine maleate, doxylamine succinate, promethazine, clindamycin phosphate, neostigmine bromide, homatropine methylbromide, bitolterol mesylate, ephedrine, phenylpropanolamine, timolol maleate, codeine phosphate, phenobarbital sodium, phenytoin sodium, sodium valproate, sodium acyclovir, dextroamphetamine, amylobarbitone sodium, butabarbital sodium, colchicine or pralidoxine chloride.

## Patentansprüche

1. Darreichungsform für eine saure oder basische Arzneimittelformulierung umfassend eine innere Membran, die die Arzneimittelformulierung umgibt, eine. Verdrängungszusammensetzung, die in Kontakt mit der inneren Membran ist und eine äußere semipermeable Membran, die die innere Membran und die Verdrängungszusammensetzung umgibt, bei der die äußere semipermeable Membran wenigstens einen Austrittsdurchgang bildet; wobei die innere Membran eine pH-sensitive Polymermembran umfaßt, die für wässerige oder biologische Flüssigkeiten durchlässig ist und die sich in Präsenz von entweder (i) einer wässerigen sauren Lösung einer Arzneimittelformulierung, wenn die Darreichungsform eine saure Arzneimittelformulierung enthält oder (ii) einer wässerigen basischen Lösung einer Arzneimittelformulierung, wenn die Darreichungsform eine basische Arzneimittelformulierung enthält, auflöst, und bei der die Verdrängungszusammensetzung im Einsatz eine wässerige oder biologische Flüssigkeit in sich aufnimmt, wobei sie sich durch die Verdrängung des freigesetzten Arzneimittels über die innere Membran durch den Austrittsdurchgang in die äußere Membran hindurch ausbreitet.

2. Darreichungsform nach Anspruch 1, bei der sich die innere Membran in der Präsenz einer sauren Lösung einer Arzneimitteiformuiierung auflöst.

3. Darreichungsform nach Anspruch 1, bei der sich die innere Membran in der Präsenz einer basischen Lösung einer Arzneimittelformulierung auflöst.

4. Darreichungsform nach Anspruch 1, bei der die äußere Membran einen Cellulosepolymer und die innere Membran einen Nicht-Cellulosepolymer umfaßt.

5. Dosierungsform nach Anspruch 4, bei der die innere Membran einen Polymer, ausgewählt aus der Gruppe bestehend aus Poly(methacrylat), Poly(methacrylsäuremethylmethacrylat), Poly(2-vinylpyridin), Poly(4-Vinylpyridin), Poly(N-pyrrolidonylethylen), Poly(iminoadipolyimeno-trimethylen-1 4-phenylintrimethylen, Poly(iminoadipolyimenotrimethylen-1,4-phenylin-posphindose), Poly(diethylcarbodimid), Poly(1,4-piperazindiylcarbonyl-ethylen-1,4-phenylenphosphinyliden), Poly(1,4-phenylenethylencarbonyliden); und Poly(2-vinylpyridin-co-stryol), umfaßt.

6. Darreichungsform nach Anspruch 5, bei dem das Arzneimittel ausgewählt wird, aus, der Gruppe bestehend aus Venlafaxinhydrochlorid, Clindamycinhydrochlorid, Tramadolhydrochlorid, Oxybutyninhydrochlorid, Oxtriphyllinhydrochlorid, Tripelenaminhydrochlorid, Tripelenamincitrat, Oxytetracyclinhydrochlorid, Tetracyclinhydrochlorid, Metronidazolhydrochlorid, Oxyphenoniumhydrobromid, Phenoxybenzamin-enitabaspalmitate, Aminocapronsäure, Disopyramidhydrosulfat, Hydrophosphat, Verapamilhydrochlorid, Papaverintartrat, Fluphenazinhydrochlorid, Ascorbinsäure, Prochlorperazinedisylat, Zitronensäure, Fluphenazinedelat, Tetracyclinfumarat, Hydroxyzinlactat, Benzaquinamidstearat, Thiaminascorbat, Ozatadinmaleat, Chlorpheniraminmaleat, Doxylaminsuccinat, Promethazin, Clindamycinphosphat, Neostigminbromid, Homatropinmethylbromid, Bitolterolmesylat, Ephedin, Phenylpropanolamin, Timololmaleat, Codeinphosphat, Phenobarbitalnatrium, Phenytoinnatrium, Natriumvalproat, Natriumacyclovir, Dextroamphetamin, Amylobarbitonnatrium, Butabarbitalnatrium, Colchicin oder Pralidoxinchlorid.

## Revendications

1. Forme posologique comprenant une formulation de médicament acide ou basique, une membrane interne entourant la formulation de médicament, une composition de déplacement en contact avec la membrane interne et une membrane semi-perméable externe entourant la membrane interne et la composition de déplacement, dans laquelle membrane semi-perméable externe est formé au moins un passage de sortie ; la membrane interne comprenant une membrane polymère sensible au pH qui est perméable au fluide aqueux ou biologique et se dissout en présence soit (i) d'une solution de formulation de médicament acide aqueuse dans le cas où la forme posologique comprend une formulation de médicament acide, soit (ii) d'une solution de formulation de médicament basique aqueuse dans le cas où la forme posologique comprend une formulation de médicament basique ; et la composition de déplacement lors de l'utilisation s'imbibant d'un fluide aqueux ou biologique, se dilatant ainsi pour déplacer le médicament libéré par la membrane interne à travers le passage de sortie dans la membrane externe.

2. Forme posologique selon la revendication 1, dans laquelle la membrane interne se dissout en présence d'une solution de formulation de médicament acide.

3. Forme posologique selon la revendication 1, dans laquelle la membrane interne se dissout en présence d'une solution de formulation de médicament basique.

4. Forme posologique selon la revendication 1, dans laquelle la membrane externe comprend un polymère cellulosique et la membrane interne comprend un polymère non cellulosique.

5. Forme posologique selon la revendication 4, dans laquelle la membrane interne comprend un polymère choisi dans le groupe constitué par les poly(méthacrylates), le poly(acide méthacrylique méthacrylate de méthyle), la poly(2-vinylpyridine), la poly(4-vinylpyridine), le poly(N-pyrrolidonyléthylène), le poly(iminoadipolyliméno-triméthylène-1,4-phénylinetriméthylène), le poly(iminoadipolyliméno-triméthylène-1,4-phényline-phosphinedose) poly(diéthylcarbodimide), le poly(1,4-pipérazinediylcarbonyl-éthylène-1,4-phénylènephosphinylidène), le poly(1,4-phénylènethylènecarbonylidène) et le poly(2-vinylpyridine-co-styrène).

6. Forme posologique selon la revendication 5, dans laquelle le médicament est choisi dans le groupe constitué par le chlorhydrate de venlafaxine, le chlorhydrate de clindamycine, le chlorhydrate de tramadol, le chlorhydrate d'oxybutynine, le chlorhydrate d'oxtriphylline, le chlorhydrate de tripélennamine, le citrate de tripélennamine, le chlorhydrate d'oxytétracycline, le chlorhydrate de tétracycline, le chlorhydrate de métronidazole, le bromhydrate d'oxyphénonium, le palmitate de phénoxybenzamine énitabas, l'acide aminocaproïque, le disopyramide hydrosulfate, hydrophosphate, le chlorhydrate de vérapamil, le tartrate de papavérine, le chlorhydrate de fluphénazine, l'acide ascorbique, l'édisylate de prochlorpérazine, l'acide citrique, l'édélate de fluphénazine, le fumarate de tétracycline, le lactate d'hydroxyzine, le stéarate de benzaquinamide, l'ascorbate de thiamine, le maléate d'ozatadine, le maléate de chlorphéniramine, le succinate de doxylamine, la prométhazine, le phosphate de clindamycine, le bromure de néostigmine, le méthylbromure d'homatropine, le mésylate de bitoltérol, l'éphédrine, la phénylpropanolamine, le maléate de timolol, le phosphate de codéine, le phénobarbital sodique, la phénytoïne sodique, le valproate de sodium, l'acyclovir sodique, la dextroamphétamine, l'amylobarbitone sodique, le butabarbital sodique, la colchicine ou le chlorure de pralidoxine.
